# FASCICULE DE BREVET EUROPEEN

(11) **EP 3 164 408 B1**
(45) Date de publication et mention de la délivrance du brevet: **12.09.2018**
(21) Numéro de dépôt: 15733755.1
(22) Date de dépôt: 02.07.2015
(51) Int. Cl.: C07C 29/68, C07C 67/08, C07F 9/40

(54) **NOUVEAU PROCÉDÉ DE FABRICATION DU (E,Z)-7,9 DODÉCANDIÉNYL-1-ACÉTATE**
NEUARTIGES VERFAHREN ZUR HERSTELLUNG VON (E,Z)-7,9-DODECADIENYL-1-ACETAT
NOVEL METHOD FOR PRODUCING (E,Z)-7,9 DODECADIENYL-1-ACETATE

(30) Priorité: 02.07.2014 FR 1456322
(43) Date de publication de la demande: 10.05.2017
(73) Titulaire: Melchior Material And Life Science France, 64170 LACQ (FR)
(72) Inventeur: DUFOUR, Samuel, F-64300 Orthez (FR); GUERRET, Olivier, F-46170 Pern (FR)
(74) Mandataire: Regimbeau
(86) Numéro de dépôt international: PCT/EP2015/065152
(87) Numéro de publication internationale: WO 2016/001383

(56) Documents cités:
- US-A- 4 912 253
- GÉRARD CAHIEZ ET AL: "Efficient Preparation of Terminal Conjugated Dienes by Coupling of Dienol Phosphates with Grignard Reagents under Iron Catalysis", ORGANIC LETTERS, vol. 10, no. 12, 1 juin 2008 (2008-06-01), pages 2389-2392, XP055179257, ISSN: 1523-7060, DOI: 10.1021/ol800816f

## Description

La présente invention concerne un procédé d'obtention du (E,Z)-7,9-dodécandiényl-1-acétate, phéromone sexuelle de l'eudemys de la vigne, ou *lobesia botrana,* un lépidoptère ravageur de la vigne. Ce procédé est caractérisé par l'utilisation d'un nouvel intermédiaire 1 défini comme un mélange des isomères de formule générale : 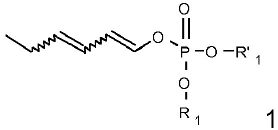 où R1 et R'1, identiques ou différents, désignent un groupement alkyle ou aryle. Ces composés sont des alkyl-aryl, dialkyl ou diaryl -hexa-1,3-dièn-1-yl phosphate.

Pour des raisons de santé publique et de gestion du potentiel agricole des sols, les technologies de traitement des cultures contre les ravageurs évoluent vers des modes d'action plus ciblés et plus respectueux de l'environnement. A ce titre, l'usage de phéromones sexuelles pour modifier le comportement d'insectes présente des atouts puisque ces phéromones sont spécifiques de chaque espèce de ravageurs et sont efficaces, à des doses très faibles, dans divers types de stratégie (piégeage, confusion sexuelle par exemple).

Cependant un frein au développement de ces technologies réside dans le coût d'accès aux molécules actives. En effet ces molécules ont souvent de nombreux isomères possibles et les technologies de synthèse sélectives sont généralement coûteuses.

Le composant principal de la phéromone sexuelle de l'eudemys de la vigne est le (E,Z)-7,9-dodécandiényl-1-acétate. Cette molécule est porteuse de 2 doubles liaisons et présente donc 4 isomères géométriques possibles rappelés dans le tableau suivant :

**Tableau 1 : isomères géométriques du 7,9-dodécandienyl-1-acétate**

| | |
|---|---|
| 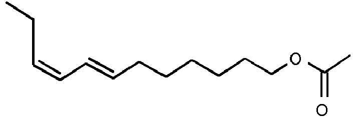 | (E,Z)-7,9-dodécandiényl-1-acétate |
| 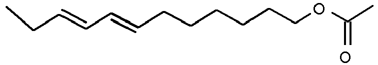 | (E,E)-7,9-dodécandiényl-1-acétate |
| 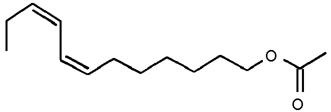 | (Z,Z)-7,9-dodécandiényl-1-acétate |
| 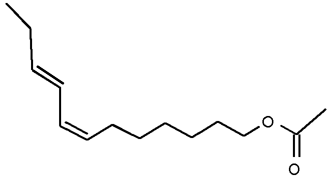 | (Z,E)-7,9-dodécandiényl-1-acétate |

Pour répondre efficacement au problème économique posé par la synthèse de cette phéromone, il convient de prendre en compte les éléments suivants :
- Seul l'isomère (E,Z) est actif. Il est donc essentiel de pouvoir le préparer majoritairement. Parmi tous ces isomères, l'isomère thermo-dynamiquement le plus stable est l'isomère (E,E). Il a été montré que lorsque le (E,Z)-7,9 dodécandiényl-1-acétate est soumis à une exposition lumineuse ou à des générateurs de radicaux libres, la molécule se réarrange en un mélange de ces isomères dans des proportions 14/70/14/2 qui reflètent l'équilibre entre les différents isomères.(Ideses & al. Journal of Chemical Ecology, VoL 8, No. 1, 1982, p. 973*).*
- Il faut cependant noter que les 3 isomères autres que le (E,Z) sont connus comme ne gênant pas l'attractivité de la phéromone (Ideses & al. Journal of Chemical Ecology, VoL 8, No. 1, 1982, p. 195).
- L'isomère (E,E) est l'isomère le plus stable et constitue l'impureté inactive principale de toutes les synthèses connues.

On comprend donc qu'un procédé de synthèse de la phéromone conduisant à l'isomère actif très pur, mais à des coûts de production très élevés, ne répondra pas à la problématique technico-économique puisqu'un procédé conduisant à une proportion plus faible du bon isomère, mais avec un bon rendement global et un nombre d'étapes de synthèse réduit, pourra avoir une meilleure efficacité économique et donc permettra un meilleur développement de la technologie de contrôle des populations de *lobesia botrana* dans les vignes par confusion sexuelle.

Pour déterminer l'efficacité économique d'une synthèse, il faut donc non seulement prendre en compte le rendement global de la synthèse qui traduit l'économie en termes de matière première mais aussi le nombre d'étapes de synthèse qui gouverne le coût de mise en oeuvre de ladite synthèse. Par étape de synthèse, la demanderesse comprend toute opération de chimie aboutissant à l'isolement d'un intermédiaire. Moins le nombre d'étapes sera grand, plus la voie de synthèse sera économique.

L'examen des solutions proposées dans l'art antérieur conduit aux résultats suivants :
Dans US 3954818 les auteurs décrivent une synthèse en plus de 9 étapes avec un rendement non précisé et une pureté de la phéromone proche de 99%. Cependant, il faut noter que ce procédé est difficilement envisageable industriellement du fait des réactifs utilisés (fil de lithium, butyl lithium, disiamylborane...). L'intermédiaire clé de cette synthèse est le méthyl non-4-én-6-ynoate.

Dans EP 3845108, le procédé décrit consiste en 8 étapes de synthèse pour un rendement global de 30% à partir de la quatrième étape et une pureté finale de 70% seulement. Le procédé est caractérisé par un intermédiaire iminophosphonate et l'utilisation de réactifs inutilisables industriellement (oxyde de mercure).

Dans EP0241335, les auteurs décrivent un procédé en 5 étapes de synthèse avec un rendement global de 10% environ. La pureté de la phéromone est de 75% au moins. L'intermédiaire clé est un 1-halogéno-(E,Z)-7,9-dodécadiène. Ce procédé coûteux nécessite des équipements d'hydrogénation sous pression. Il utilise aussi une réaction de Wittig qui génère des quantités importantes d'oxyde de triphényl phosphine coûteuses à éliminer.

Le brevet US 4912253 décrit la synthèse de la phéromone de l'eudemys de la vigne par un couplage cataysé au cuivre entre un magnésien (dérivé du chloropentanol) et l'acéate de (E,Z)-2,4-heptadiényle. La préparation du dérivé acétate est cependant difficile et cette voie d'accès bien que convergente reste coûteuse.

Dans FR 2609868, les auteurs rapportent deux procédés de synthèse d'un précurseur d'un analogue de la phéromone (le Z-9- dodécèn-9-ynol) via un procédé caractérisé en ce que l'intermédiaire clé est un alcynol protégé par une fonction tétrahydropyranyl. Les rendements sont similaires à ceux de EP 0241335.

Dans US7932410, une méthode générale pour former des diènes conjugués à longue chaine grasse est décrite, et est caractérisée par l'utilisation d'esters encombrés en position alpha d'une double liaison tels que l'isobutyrate de 1-pentèn-3-yl qui est couplé à un réactif de Grignard via une catalyse à base de complexes du cuivre. Cette méthode n'est pas applicable industriellement à la synthèse du (E,Z)-7,9 dodécandiényl-1-acétate car l'isobutyrate de 1,3-hept-dièn-3-yl nécessaire pour cette synthèse est très difficilement accessible industriellement.

Les méthodologies classiques de synthèse connues dans la littérature pour la synthèse de la phéromone de l'eudemys de la vigne utilisent donc soit des intermédiaires de type acétylénique (couplage via des acétylures), soit des réactions de couplage entre aldéhyde et ylures de phosphore dites réactions de Wittig.

D'autres voies plus originales ont aussi été rapportées.

Dans Alexakis & al. Tetrahedron, vol. 45, n°2, p. 389, 1989, les auteurs décrivent un procédé en 8 étapes qui exploite la réactivité des fonctions époxydes en présence de dérivés du silicium.

Dans Loreau & al. Chemistry and Physics, 110 (2001) p. 57, une voie de synthèse sélective en 7 étapes, est décrite. Elle présente un coût élevé du fait des catalyseurs au palladium qu'elle emploie.

Dans Krishnam & al, Journal of Agric. & Food chemistry, vol 50, 22, 2002, p.6366, les auteurs développent une synthèse en 5 étapes mais comprenant l'emploi de catalyseurs inexploitables industriellement.

Dans Franke & al. Znatursforch vol 57 p. 739 (2002) les auteurs rapportent une voie de synthèse, basse température, utilisant le bis(triméthylsilyl)amidure de sodium qui est difficilement extrapolable.

Toutes ces synthèses ont des rendements faibles et présentent un nombre d'étapes trop important pour être facilement industrialisables. En général, les problématiques de sélectivité sont réglées par un post-traitement du brut réactionnel via des technologies connues de l'homme du métier pour séparer les isomères géométriques. Citons à titre d'exemple, la chromatographie sur colonne de silice imprégnée au sel d'argent ou encore le piégeage par réaction de Diels Alder avec du tétracyanoéthylène des composés E,E ou enfin la complexation préférentielle dans une matrice d'urée des composés E,E. Ces étapes supplémentaires sont nécessaires si les sélectivités des méthodes de synthèse sont mauvaises.

Cet état de l'art montre bien que réduire le nombre d'étapes de synthèse tout en maximisant la sélectivité de la synthèse du (E,Z)-7,9 dodécandiényl-1-acétate permettrait d'une part des économies de matières premières via un meilleur rendement et des économies de coûts de production réduisant le temps nécessaire à la production.

La demanderesse a donc trouvé un nouveau procédé en deux étapes de synthèse permettant d'accéder au (E,Z)-7,9-dodécandiényl-1-acétate avec d'excellents rendements et une sélectivité supérieure à 70%.

Ce procédé nouveau est caractérisé en ce qu'on transforme le 2-hexénal, produit facilement accessible, selon une première étape A, en intermédiaire 1, nouveau, qui est lui-même ensuite transformé en (E,Z)-7,9-dodécandiényl-1-acétate brut, via une seconde étape B, selon le schéma global de synthèse suivant : 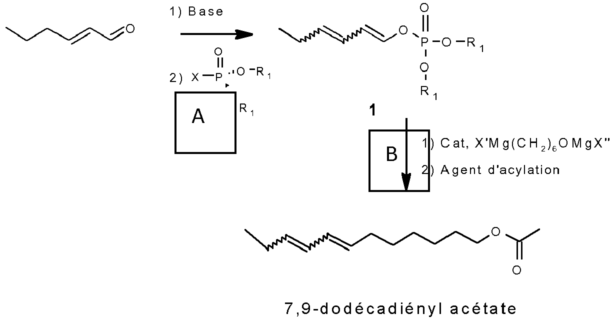 et qu'éventuellement, on purifie le (E,Z)-7,9-dodécandiényl-1-acétate par des méthodes connues de l'homme du métier.

Ainsi, la présente invention vise un composé de formule générale 1 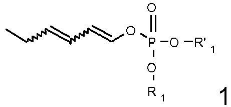 où R1 et R'1 identiques ou différents désignent un groupement alkyle ou aryle.

Dans un mode de réalisation particulier, l'invention vise un composé 1 dans lequel le groupement alkyle est choisi parmi les alkyles en C1-C6, linéaires ou ramifiés et le groupement aryle est choisi parmi phényle, benzyle, mésityle, ou tolyle. Le groupement alkyle en C1-C6 linéaire ou ramifié peut être choisi parmi méthyle, éthyle, propyle, iso-propyle, butyle, n-butyle, sec-butyle, iso-butyle, tert-butyle, pentyle, hexyle.

L'invention concerne aussi l'utilisation d'un composé 1 selon l'invention pour la synthèse de composés comprenant un motif diénique conjugué de type : 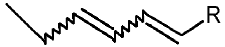 où R représente le radical du composé sur lequel est greffé le motif diénique conjugué. Le radical R du composé sur lequel est greffé le motif diène conjugué peut être de nature variée, hydrocarboné ou carboné par exemple, et de manière générale tout radical ou motif sur lequel on souhaite greffer le motif diénique conjugué supra en question.

De manière préférentielle, la présente invention concerne l'utilisation d'un composé 1 selon la présente invention pour la synthèse de phéromones comportant au moins un radical conjugué de formule :

CH₃-CH₂-CH=CH=CH-

Selon un mode de réalisation particulier, l'invention concerne l'utilisation d'un composé 1 selon l'invention comme intermédiaire pour la synthèse du (E,Z)-7,9-dodécènyl acétate.

Ainsi, un objet de la présente invention, à savoir l'étape A visée plus haut, concerne, un procédé de préparation du composé 1 comprenant :
- la fourniture de 2-hexénal dans un solvant approprie S1,
- l'ajout d'une base forte peu nucléophile à une température T1 comprise entre -78°C et 25°C afin de former l'énolate,
- l'ajout, à une température T2, identique ou différente de T1, comprise entre - 78°C et 25°C d'un halogénophosphate de formule

   X-P(O)(OR1)(OR1')

   où X est un halogène et R1 et R'1 désignent des groupes identiques ou différents choisis parmi un alkyl en C1-C6 linéaire ou ramifié, un aryle tel que phényle, benzyle, mésityle ou tolyle,
- la récupération du composé 1 après lavage et séchage de la phase organique.

Le procédé de préparation du composé 1 selon l'invention, est plus précisément décrit par le protocole suivant :
a) On prépare, dans un réacteur sous agitation, un équivalent de 2-hexénal dilué dans 2 à 60 volumes de solvant ou d'un mélange de solvants S1. La température du milieu réactionnel est amenée à une température T1 comprise entre -78°C et 25°C, on ajoute ensuite 1 à 2 équivalents d'une base forte peu nucléophile comme un alcoolate encombré, un amidure encombré, une amidine.
b) On ajoute ensuite au milieu réactionnel, à une température T2 comprise entre -78°C et 25°C, éventuellement identique à T1, l'halogénophosphate d'alkyl ou d'aryl de formule générale :

   XP(O)(OR₁)(OR'₁)
où R1, R'1 identiques ou différents sont des groupements alkyles en C1-C6 linéaires ou ramifiés ou aryles et X un atome d'halogène, de préférence le chlore. L'halogénophosphate est ajouté à raison de 1 à 2 équivalents par rapport au 2-hexénal. Le groupement alkyle en C1-C6 linéaire ou ramifié peut être choisi parmi méthyle, éthyle, propyle, iso-propyle, butyle, n-butyle, sec-butyle, iso-butyle, tert-butyle, pentyle, hexyle. Le groupement aryle est choisi parmi phényle, benzyle, mésityle, ou tolyle

On maintient sous agitation à la température T2, le temps que le composé 1 soit formé.

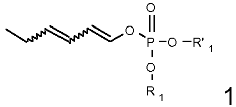

Ce produit est en fait un mélange des isomères géométriques (E,E), (E,Z), (Z,E) et (Z,Z), dont le ratio des isomères détermine celui de l'ensemble du procédé.

Dans un mode de réalisation préféré, le procédé de synthèse du composé 1 selon l'invention est caractérisé en ce que le solvant S1 est choisi dans le groupe constitué par le tétrahydrofurane (THF), le méthyl tétrahydrofurane (MeTHF), la tétraméthyléthylènediamine (TMEDA), le tétrahydropyrane, le diméthoxyéthane (DME), le diéthyl éther, le méthyl-tertiobutyl éther (MTBE), des solvants azotés très polaires comme le diméthylformamide (DMF), la *N*-méthylpyrrolidinone (NMP), la N,N'- Diméthyl propylène urée (DMPU), le méthylcyclohexane (MeCy), les alcanes de moins de 8 atomes de carbone, les solvants aromatiques tels que le toluène ainsi que leurs mélanges. Les mélanges convenables sont THF/NMP, THF/DMF, THF/DMPU, THF/TMEDA, MTBE/NMP, MeCy/NMP, par exemple.

Selon une variante particulière de l'invention, le procédé de synthèse du composé 1 ici décrit est caractérisé en ce que les températures T1 et T2 identiques ou différentes sont comprises entre -40°C et 15°C.

De manière plus particulière encore, le procédé de synthèse du composé 1 selon l'invention est caractérisé en ce que les températures T1 et T2 identiques ou différentes sont comprises entre -20°C et 0°C.

Le procédé de synthèse du composé 1 selon la présente invention est caractérisé en ce que la base forte peu nucléophile est choisie dans le groupe constitué par le terbutanolate de potassium ou de sodium, le diisopropyleamidure de sodium ou de potassium, l'hexaméthyldisilylazane de sodium ou de potassium, le 1,5-diazabicyclo[4.3.0]non-5-ène (DBN), ou le 1.8-diazabicyclo[5.4.0]undec-7-ène (DBU).

Le traitement aqueux de la solution organique sert à éliminer certains solvants contenus dans S1 avant le couplage métallique mis en oeuvre par la suite, ainsi que les sels formés par cette étape et l'excès de réactifs.

La solution obtenue est donc une solution du composé 1 dans un solvant ou un mélange de solvants S2. La phase organique est séchée pour être directement utilisée ensuite selon l'étape B mentionnée ci-avant ou évaporée sous pression réduite pour isoler l'intermédiaire 1.

Aussi, la présente invention concerne un procédé de préparation du (E,Z)-7,9-dodécandiènyl-1- acétate, représentant l'étape B indiquée supra, comprenant les étapes suivantes :
- fourniture du produit 1 dans un solvant S2,
- ajout d'un système catalytique contenant au moins un atome de fer au degré d'oxydation III,
- ajout, à une température T3 comprise entre -20° et 60°C, du composé de formule générale X'Mg-(CH₂)₆-OMgX" où X' et X" identiques ou différents désignent un atome d'halogène,
- ajout d'un agent d'acétylation,
- récupération du (E,Z)-7,9-dodécandiènyl-1-acétate après lavage et évaporation des solvants organiques.

Dans un mode de réalisation particulier, le procédé de préparation du (E,Z)-7,9-dodécandiènyl-1-acétate est caractérisé en ce que le solvant S2 est choisi dans le groupe constitué du THF, du MeTHF, du MeCy, du diéthyl éther, du méthyl terbutyl éther (MTBE), du 1,2-diméthoxyéthane et leurs mélanges. De préférence, S2 est un mélange d'éthers ou d'alcanes et de manière encore plus préférentielle, S2 est choisi dans le groupe constitué par le THF, le MeCy, le MTBE et le MeTHF.

Dans un mode préférentiel de réalisation, le procédé de préparation du 7,9-dodécandiènyl-1- acétate selon la présente invention est caractérisé en ce que la température T3 est comprise entre 0°C et 60°C, particulièrement entre 0°C et 30°C.

Le procédé de préparation du (E,Z)-7,9-dodécandiènyl-1-acétate selon la présente invention est caractérisé par le fait que le système catalytique Cat est choisi dans le groupe constitué par les trihalogénures de fer, le triacétate de fer, le triacétylacétonate de fer, le nitrate de fer (III), le phosphate de fer (III) éventuellement en présence de ligands choisis parmi les ligands phosphorés ou azotés.

Dans un mode de réalisation particulier du procédé selon la présente invention, l'agent d'acétylation est choisi dans le groupe constitué par les halogénures d'acétoyl, l'anhydride acétique et l'acétate d'alkyle.

Le procédé de préparation du (E,Z)-7,9-dodécandiènyl-1-acétate selon la présente invention, à savoir l'étape B telle qu'indiquée ci-avant, est plus précisément détaillé ci-après.
a) La solution du composé 1 dans S2 est mise en présence de 0.5%mol à 2%mol d'un système catalytique Cat qui contient un atome de Fer de degré d'oxydation III. Le mélange est maintenu sous agitation, à une température T3 comprise entre -20° et 60°C, particulièrement entre -5°C et 25°C. On ajoute ensuite une solution de solvant S3 contenant le composé de formule générale X'Mg-(CH₂)₆-OMgX " où X' et X", identiques ou différents désignent un atome d'halogène, particulièrement un atome de Chlore Cl ou de Brome Br, raison de 0.95 à 1.5 équivalent par rapport à l'hexénal. La réaction conduit à une solution de (E,Z)-7,9-dodécadiénolate.
b) On ajoute ensuite un réactif d'acétylation tel que les halogénures d'acétoyl, l'anhydride acétique ou un acétate d'alkyle à raison de 1.5 à 5 équivalents.
c) Le milieu réactionnel est ensuite lavé avec une solution aqueuse acide à un pH compris entre 2 et 6 puis avec une solution à pH compris entre 7 et 9. La phase organique est récupérée puis concentrée sous vide pour récupérer le (E,Z)-7,9-dodécandiényl-1-acétate brut.

En fonction de la pureté requise, on peut purifier le (E,Z)-7,9-dodécandiényl-1-acétate. La purification du produit se fait par une distillation sous vide.

Dans un mode de réalisation particulière le solvant S3 du composé de formule générale X'Mg-(CH₂)₆-OMgX " peut être choisi dans le groupe constitué par THF, MeTHF et leurs mélanges.

Il est possible de pousser plus loin la purification en utilisant une méthode du métier pour réduire l'importance de l'isomère (E,E). Par exemple, en utilisant la complexation préférentielle de cet isomère dans une matrice d'urée (voir Leadbetter & al. Journal of Chemical Ecology, Vol 5, No. 1, 1979 p. 101) ou par réaction préférentielle avec le tétracyano éthylène.

Dans une variante du procédé, il est possible de procéder à l'enchainement de l'étape A et de l'étape B sans isoler l'intermédiaire 1 ce qui réduit encore plus les coûts de mise en oeuvre du procédé.

La présente invention concerne ainsi un procédé de préparation du 7,9-dodécandiényl-1-acétate, en particulier le (E,Z) 7,9-dodécandiényl-1-acétate, à partir de 2-hexénal par la succession des étapes A et B.

Avec ce procédé, la demanderesse obtient des rendements et des temps de cycle de production bien meilleurs que tout ce qui est connu de l'homme du métier.

Les transferts de magnésien catalysés par des métaux sur des énols phosphate mis en oeuvre dans l'étape B du procédé sont décrits dans la littérature (voir Cahiez & al. dans J. Org. Chem. 2008, 73, 6871 et Org. Lett., Vol. 10, No. 12, 2008, 2389).

Par ailleurs, il apparaît que le composé 1 est un composé chimique nouveau accessible facilement à partir du 2-hexénal qui permet d'accéder au composant principal de la phéromone de l'eudémys de la vigne en une seule étape.

La lecture des travaux de Cahiez montre en outre que ces réactions sont très sélectives quand l'aldéhyde de départ est le 2-buténal ou le 2-penténal mais que cette sélectivité se perd pour un nombre d'atomes de carbone plus élevé que 5. Il n'était donc pas évident qu'un aldéhyde avec 6 atomes de carbone conduise à un intermédiaire de type 1 avec de bonnes sélectivités (>80%).

De même que le nouveau composé 1 permet d'accéder au (E,Z)-7,9-dodécadiényl-acétate, il permet aussi d'accéder à des dérivés diéniques de formule générale : 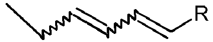 où R représente le radical du composé sur lequel est greffé le motif diénique conjugué, par exemple un radical hydrocarboné, carboné ou tout autre radical ou motif sur lequel on pourra souhaiter le greffage dudit motif diénique conjugué.

### Exemples :

Les matières premières et solvants sont les matières premières trouvées commercialement chez Sigma Aldrich.

La méthode analytique consiste en une analyse par chromatographie en phase gaz (GC) sur un appareil HP 5890 Series II équipé d'un détecteur FID. La colonne chromatographique est une colonne Innowax 30m, 0.25 mm, 0.25 µm, le gaz vecteur étant l'hélium.

Le four suit le profil de température suivant : T0=150°C, Temps initial 10 min. Gradient 20°/min ; Température finale : 200°C. Durée 7 min.

L'injecteur est à 250°C, le détecteur à 300°C.

Le volume injecté est de 1 µl. La concentration de l'échantillon est de 4 g/l dans l'acétate d'éthyle (AcOEt).

Les réactions sont réalisées dans un réacteur de 2 L en verre à double enveloppe muni d'un système de refroidissement basse température et les distillations sont effectuées au moyen d'une colonne en verre de 10 plateaux théoriques.

### Exemple 1 : Préparation de l'intermédiaire clé 1 avec R1 et R'1=éthyl :

On prépare dans le réacteur muni d'une agitation, 50 g (0.51 moles) de 2-hexénal dilué dans 10 volumes d'un mélange 3-2 de THF et de NMP (S1), la température du milieu réactionnel est descendue à une température T1 de - 15°C, on ajoute ensuite 69 g (0.61 moles) de ter-butanolate de potassium. Au bout d'une heure, on ajoute au milieu réactionnel, à la température T2, toujours de -15°C, 97g (0.56 moles) de chlorophosphate de diéthyl. La réaction est agitée pendant 1 heure puis le produit formé est isolé par lavage avec une solution de soude qui permet d'obtenir 500g d'une solution de l'énolphosphate dans le THF. La solution est séchée sur MgSO4 jusqu'à une teneur en eau résiduelle inférieure à 0.1%, la teneur en énolphosphate est dosée par chromatographie gazeuse. Cette solution pourra être utilisée telle quelle dans les exemples suivants.

L'isolement du composé 1 se fait par évaporation sous vide partiel des solvants. On récupère 105 g de diéthyl-hexa-1,3-dien-1-yl phosphate sous la forme d'un mélange de 2 isomères dans le ratio suivant :
Z,Z- diéthyl-hexa-1,3-dien-1-yl phosphate : <1%
Z,E- diéthyl-hexa-1,3-dien-1-yl phosphate :<1%
E,Z- diéthyl-hexa-1,3-dien-1-yl phosphate : 72%
E,E- diéthyl-hexa-1,3-dien-1-yl phosphate : 27%

### Caractérisation :

Temps de rétention par chromatographie phase gaz : Z,Z- diéthyl-hexa-1,3-dien-1-yl phosphate :13,96mn ; Z,E- diéthyl-hexa-1,3-dien-1-yl phosphate: 14,27mn ; E,Z- diéthyl-hexa-1,3-dien-1-yl phosphate: 14,43mn ; E,E- diéthyl-hexa-1,3-dien-1-yl phosphate : 15,27mn.

RMN ¹H(δ ppm, CDCl₃): 6,65 (1H, doublet de doublet, CH); 6,26 (1H, triplet, CH); 5,79 (1H, triplet, CH); 5,4 (1H, doublet de triplets), 4,15 (4H, triplet, OCH₂), 2,11 (2H, massif, CH₃C*H*₂), 1,33 (6H, triplet, C*H*₃CH₂O), 0,97 (3H, triplet, C*H*₃CH₂CH).

### Exemple 2 : synthèse du (E,Z) 7,9-dodécandiényl-1-acétate.

On ajoute à la solution intermédiaire de l'exemple 1, 1,8g (5.1) mmol de tri(acétylacétonate) de fer puis on coule doucement dans le réacteur 0.61 moles de BrMg-(CH₂)₆-OMgBr en solution à 1.6 mol/L dans le MeTHF. Pendant cette addition, on maintient le milieu réactionnel à la température T3 de 25°C. Après deux heures, on ajoute 260 g d'anhydride acétique au milieu réactionnel, qui est maintenu sous agitation à température ambiante, jusqu'à conversion totale de l'alcoolate formé lors du couplage au fer.

Le milieu réactionnel est ensuite lavé avec une solution d'acide chlorhydrique 0.01 molaire, puis avec une solution de carbonate de sodium à un pH de 8. La phase organique est récupérée puis concentrée sous vide pour récupérer le 7,9-dodécandiényl-1-acétate brut (88g dosé à 90% de pureté chimique et dans un rapport des isomères E,Z/Z,Z de 76%). Soit un rendement brut de 69% par rapport à l'hexénal.

Le brut est distillé sous vide poussé pour conduire à 75g de 7,9-dodécandiényl-1-acétate avec 98% de pureté chimique et une teneur en isomère E,Z de 76%. Soit un rendement de 66% par rapport à l'hexénal.

### Enrichissement en l'isomère E,Z :

Les 75 g obtenus sont mélangés à 130g d'urée dans 800ml de méthanol. Le mélange est laissé au repos pendant 3 heures. La suspension est filtrée et le résidu est lavé, deux fois, avec 100 ml de diéthyl éther. Les fractions de lavages sont rassemblées avec le filtrat puis évaporées sous pression réduite jusqu'à évaporation complète des solvants. On obtient, 61g de 7,9-dodécandiényl-1-acétate d'une pureté chimique de 98% et avec une teneur de 90% en l'isomère E,Z.

### Exemples 3 à 20 :

Dans tous les exemples suivants, on utilise le protocole expérimental des exemples 1 et 2 en faisant varier les paramètres suivants :
- S1 : solvant de dilution de l'hexénal.
- S2 : solvant de dilution diéthyl-hexa-1,3-dien-1-yl-phosphate.
- S3 : solvant de dilution du composé magnésien
- X' : halogénure du composé X'Mg-(CH₂)₆-OMgX'
- T1 : température de déprotonation de l'hexénal
- T2 : température de synthèse du diéthyl-hexa-1,3-dien-1-yl-phosphate
- T3 : température du couplage organomagnésien
- N1 = nombre de moles de terbutanolate de potassium/nombre de moles d'hexénal
- N2 = nombre de moles de chlorophosphate de diéthyl/nombre de moles d'hexénal
- N3 = nombre de moles de magnésien/nombre de moles d'hexénal

Les paramètres variant dans les exemples sont résumés dans le tableau 1

| Exemple | S1* | S2 | S3 | X' | T1 | T2 | T3 | N2 (mol) | N4 (mol) | N4 (mol) |
|---|---|---|---|---|---|---|---|---|---|---|
| 3 | THF/NMP (17/12) | THF | THF | Br | -78 | -78 | 0 | 1,2 | 1,1 | 1,2 |
| 4 | THF/NMP (36/12) | THF | MeTHF | Br | -70 | -78 | 0 | 1,2 | 1,2 | 1,2 |
| 5 | THF/NMP (36/17) | THF | MeTHF | Br | -55 | -78 | 0 | 1,1 | 1,1 | 1,2 |
| 6 | THF/DMPU (36/17) | THF | MeTHF | Br | -35 | -78 | 0 | 1,2 | 1,1 | 1,2 |
| 7 | THF/NMP (36/17) | THF | MeTHF | Br | -25 | 0 | 25 | 1,2 | 1,1 | 1,2 |
| 8 | THF/NMP (36/17) | THF | MeTHF | Br | -15 | 0 | 0 | 1,2 | 1,1 | 1,2 |
| 9 | THF/NMP (36/17) | THF | MeTHF | Br | -15 | -15 | 0 | 1,2 | 1,1 | 1,2 |
| 10 | THF/DMPU (36/17) | THF | MeTHF | Br | -15 | -15 | 0 | 1,2 | 1,1 | 1,2 |
| 11 | THF/TMEDA (36/17) | THF | MeTHF | Br | -15 | -15 | 0 | 1,2 | 1,1 | 1,2 |
| 12 | THF/NMP (36/17) | THF | MeTHF | Br | -15 | -10 | 0 | 1,2 | 1,1 | 1,2 |
| 13 | THF/NMP (36/17) | THF | MeTHF | Br | -15 | 0 | 0 | 1,2 | 1,1 | 1,2 |
| 14 | MTBE/NMP (36/17) | MTBE | MeTHF | Br | -15 | -15 | 25 | 1,2 | 1,1 | 1,2 |
| 15 | MeCy/NMP (36/17) | MeCy | MeTHF | Br | -15 | -15 | 0 | 1,2 | 1,1 | 1,2 |
| 16 | MeCy/NMP (10/2) | MeCy | MeTHF | Br | -15 | -15 | 25 | 1,2 | 1,1 | 1,2 |
| 17 | THF/DMF (36/12) | THF | MeTHF | Br | -15 | -15 | 25 | 1,2 | 1,1 | 1,2 |
| 18 | DMF/NMP (14/2) | THF | MeTHF | Br | -15 | -15 | 30 | 1,2 | 1,1 | 1,2 |
| 19 | DMF/NMP (14/2) | THF | THF | Br | -15 | -15 | 25 | 1,2 | 1,1 | 1,2 |
| 20 | DMPU/NMP (14/2) | THF | THF | Br | -10 | -10 | 20 | 1,2 | 1,1 | 1,2 |
| 21 | DMPU/NMP (2/2) | THF | THF | Br | -10 | -10 | 25 | 1,2 | 1,1 | 1,2 |
| 22 | NMP 5V | THF | THF | | -5 | -5 | 25 | 1,2 | 1,1 | 1,2 |
| 23 | NMP 5V | THF | THF | | -5 | 0 | 25 | 1,2 | 1,1 | 1,2 |

| | | | | | | | | | | |
|---|---|---|---|---|---|---|---|---|---|---|
| **le mélange de solvant est indiqué : les chiffres entre parenthèses désignent les volumes respectifs des composants du solvant par rapport à l'hexénal.* | | | | | | | | | | |

Le rendement brut (Rb) de chaque exemple traduit le rendement molaire brut avant distillation.

Le ratio Rb_{(E,Z)} désigne la proportion de l'isomère E,Z par rapport à l'isomère E,E.

Les données Rd et Rd_{(E,Z)} désignent respectivement le rendement molaire, la proportion de l'isomère E,Z par rapport à l'isomère E,E après l'étape de purification.

Ces résultats pour chaque exemple sont résumés dans le tableau 2 :

| Exemple | Rb (%) | Rb_{(E,Z)} (%) | Rd (%) | Rd_{(E,Z)} (%) |
|---|---|---|---|---|
| 3 | 63 | 80 | 61 | 85 |
| 4 | 62 | 80 | 60 | 85 |
| 5 | 59 | 79 | 50 | 86 |
| 6 | 61 | 74 | 51 | 86 |
| 7 | 59 | 77 | 51 | 82 |
| 8 | 61 | 74 | 52 | 81 |
| 9 | 59 | 74 | 49 | 81 |
| 10 | 49 | 57 | ** | ** |
| 11 | 42 | 66 | ** | ** |
| 12 | 59 | 74 | 24 | 91 |
| 13 | 44 | 63 | ** | ** |
| 14 | 51 | 74 | ** | ** |
| 15 | 62 | 74 | 49 | 81 |
| 16 | 44 | 65 | ** | ** |
| 17 | 65 | 74 | 52 | 80 |
| 18 | 14 | 72 | ** | ** |
| 19 | 14 | 72 | ** | ** |
| 20 | 38 | 66 | ** | ** |
| 21 | 38 | 66 | ** | ** |
| 22 | 25 | 57 | ** | ** |
| 23 | 25 | 57 | ** | ** |

| | | | | |
|---|---|---|---|---|
| ** *les expériences n'ont pas été purifiées* | | | | |

## Revendications

1. Composé de formule générale 1 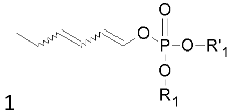 où R1 et R'1 identiques ou différents désignent un groupement alkyle ou aryle.

2. Composé selon la revendication 1 dans lequel le groupement alkyl est choisi parmi les alkyles en C1-C6, linéaires ou ramifiés, le groupement aryl est choisi parmi phényle, benzyle, mésityle, ou tolyle.

3. Utilisation d'un composé 1 selon l'un des revendications 1 à 2, pour la synthèse de composé diénique de formule générale : 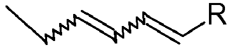 où R représente le radical du composé sur lequel est greffé le motif diénique conjugué.

4. Utilisation d'un composé 1 selon l'une des revendications 1 à 2, pour la synthèse de phéromones comportant au moins un radical conjugué de formule :
CH₃-CH₂-CH=CH=CH-

5. Utilisation d'un composé 1 selon l'une des revendications 1 à 2, comme intermédiaire pour la synthèse de (E,Z)-7,9-dodécadiènyl-1-acétate.

6. Procédé de préparation de composé 1 comprenant les étapes suivantes :
- fourniture de 2 hexénal dans un solvant approprie S1,
- ajout d'une base forte peu nucléophile à une température T1 comprise entre -78°C et 25°C afin de former l'énolate,
- ajout, à une température T2, identique ou différente de T1, comprise entre -78°C et 25°C d'un halogénophosphate de formule :
X-P(O)(OR1)(OR1')
où X est un halogène et R1 et R'1 désignent des groupes identiques ou différents choisis parmi un alkyl en C1-C6 linéaire ou ramifié, un aryle tel que phényle, benzyle, mésityle ou tolyle,
- récupération du composé 1 après lavage et séchage de la phase organique.

7. Procédé selon la revendication 6 **caractérisé en ce que** le solvant S1 est choisi dans le groupe constitué par le tétrahydrofurane (THF), le méthyl tétrahydrofurane (MeTHF), la tétraméthylethylènediamine (TMEDA) le tétrahydropyrane, le diméthoxyéthane (DME), le diéthyl éther, le méthyl-tertiobutyl éther, des solvants azotés très polaires comme le diméthylformamide (DMF), la *N*-méthylpyrrolidinone (NMP), la N,N'- Diméthyl propylène urée (DMPU), le méthylcyclohexane (MeCy) les alcanes de moins de 8 atomes de carbone, les solvants aromatiques tels que le toluène ainsi que leurs mélanges.

8. Procédé selon l'une des revendications 6 ou 7, **caractérisé en ce que** les températures T1 et T2 identiques ou différentes sont comprises entre
-40°C et 15°C.

9. Procédé selon les revendications 6 à 8, **caractérisé en ce que** les températures T1 et T2 identiques ou différentes sont comprises entre -20°C et 0°C.

10. Procédé selon les revendications 6 à 9, **caractérisé en ce que** la base forte peu nucléophile est choisie dans le groupe constitué par le terbutanolalte de sodium ou de potassium, le diisopropyleamidure de sodium ou de potassium, l'hexaméthyldisilylazane de sodium ou de potassium, le 1,5-diazabicyclo[4.3.0]non-5-ène (DBN), ou le 1.8-diazabicyclo[5.4.0]undec-7-ène (DBU).

11. Procédé de préparation du (E,Z)-7,9-dodécandiènyl-1-acétate, comprenant les étapes suivantes :
- fourniture du produit 1 dans un solvant S2,
- ajout d'un système catalytique contenant au moins un atome de fer au degré d'oxydation III,
- ajout, à une température T3 comprise entre -20° et 60°C, du composé de formule générale X'Mg-(CH₂)₆-OMgX" où X' et X" identiques ou différents désignent un halogène,
- ajout d'un agent d'acétylation,
- récupération du (E,Z)-7,9-dodécandiènyl-1-acétate après lavage et évaporation des solvants organiques.

12. Procédé selon la revendication 11, **caractérisé en ce que** le solvant S2 est choisi dans le groupe constitué du THF, du MeTHF, du méthylcyclohexane (MeCy), du diéthyl éther, du méthyl terbutyl éther, du 1,2-diméthoxyéthane et de leurs mélanges.

13. Procédé selon les revendications 11 et 12 **caractérisé en ce que** la température T3 est comprise entre 0°C et 60°C.

14. Procédé selon les revendications 11 à 13 **caractérisé en ce que** le système catalytique Cat est choisi dans le groupe constitué par les trihalogénures de fer, le triacétate de fer, le triacétylacétonate de fer, le nitrate de fer (III), le phosphate de fer (III), éventuellement en présence de ligand choisi parmi les ligands phosphorés ou azotés.

15. Procédé selon l'un des revendications 1 à 14, **caractérisé en ce que** X' et X" identiques ou différents désignent un halogène choisi parmi le chlore ou le brome, et l'agent d'acétylation est choisi parmi les halogénures d'acétoyl, l'anhydride acétique et l'acétate d'alkyle.

## Patentansprüche

1. Verbindung der allgemeinen Formel 1 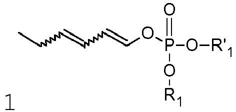 wobei R1 und R'1 gleich oder verschieden eine Alkyl- oder Arylgruppe bedeuten.

2. Verbindung nach Anspruch 1, wobei die Alkylgruppe aus linearen oder verzweigten C1-C6-Alkylen ausgewählt ist, die Arylgruppe aus Phenyl, Benzyl, Mesityl oder Tolyl ausgewählt ist.

3. Verwendung einer Verbindung 1 nach einem der Ansprüche 1 bis 2 zur Synthese einer Dien-Verbindung der allgemeinen Formel: 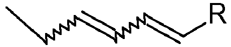 wobei R das Radikal der Verbindung darstellt, auf die die konjugierte Dien-Einheit gepfropft ist.

4. Verwendung einer Verbindung 1 nach einem der Ansprüche 1 bis 2 zur Synthese von Pheromonen, umfassend mindestens ein konjugiertes Radikal der Formel:
CH₃-CH₂-CH=CH=CH-

5. Verwendung einer Verbindung 1 nach einem der Ansprüche 1 bis 2 als Zwischenprodukt für die Synthese von (E,Z)-7,9-Dodecadienyl-1-acetat.

6. Verfahren zur Herstellung von Verbindung 1, das die folgenden Schritte umfasst:
- Zuführung von 2 Hexenal in ein geeignetes Lösungsmittel S1,
- Zusatz einer schwach nukleophilen starken Base bei einer Temperatur T1 zwischen -78 °C und 25 °C, um das Enolat zu bilden,
- Zusatz bei einer Temperatur T2, die gleich oder verschieden von T1 ist und zwischen -78 °C und 25 °C liegt, eines Halogenphosphats der Formel:
X-P(O)(OR1)(OR1')
wobei X ein Halogen ist und R1 und R'1 gleiche oder verschiedene Gruppen bezeichnen, die unter einem linearen oder verzweigten C1-C6-Alkyl, einem Aryl wie Phenyl, Benzyl, Mesityl oder Tolyl ausgewählt sind,
- Rückgewinnung der Verbindung 1 nach dem Waschen und Trocknen der organischen Phase.

7. Verfahren nach Anspruch 6, **dadurch gekennzeichnet, dass** das Lösungsmittel S1 aus der Gruppe bestehend aus Tetrahydrofuran (THF), Methyltetrahydrofuran (MeTHF), Tetramethylendiamin (TMEDA), Tetrahydropyran, Dimethoxyethan (DME), Diethylether, Methyl-tertiobutylether, hochpolaren stickstoffhaltigen Lösungsmitteln wie Dimethylformamid (DMF), *N*-Methylpyrrolidinon (NMP), N,N'-Dimethylpropylenharnstoff (DMPU), Methylcyclohexan (MeCy), Alkanen mit weniger als 8 Kohlenstoffatomen, aromatischen Lösungsmitteln wie Toluol und deren Mischungen ausgewählt ist.

8. Verfahren nach einem der Ansprüche 6 oder 7, **dadurch gekennzeichnet, dass** die gleichen oder unterschiedlichen Temperaturen T1 und T2 zwischen - 40 °C und 15 °C liegen.

9. Verfahren nach Anspruch 6 bis 8, **dadurch gekennzeichnet, dass** die gleichen oder unterschiedlichen Temperaturen T1 und T2 zwischen - 20 °C und 0 °C liegen.

10. Verfahren nach den Ansprüchen 6 bis 9, **dadurch gekennzeichnet, dass** die schwach nukleophile starke Base aus der Gruppe bestehend aus Natrium- oder Kaliumterbutanolalte, Natrium- oder Kaliumdiisopropylamidid, Natrium- oder Kaliumhexamethyldisilylazan, 1,5-Diazabicyclo [4.3.0] nicht-5-en (DBN) oder 1,8-Diazabicyclo[5.4.0]undec-7-en (DBU) ausgewählt ist.

11. Verfahren zur Herstellung von (E,Z)-7,9-Dodecandienyl-1-acetat, das die folgenden Schritte umfasst:
- Zuführung von Produkt 1 in ein Lösungsmittel S2,
- Zusatz eines katalytischen Systems, das mindestens ein Eisenatom im Oxidationsgrad III enthält,
- Zusatz bei einer Temperatur T3 zwischen -20° und 60 °C der Verbindung der allgemeinen Formel X'Mg-(CH₂)₆-OMgX" wobei X' und X" gleich oder verschieden ein Halogen bezeichnen,
- Zusatz eines Acetylierungsmittels,
- Rückgewinnung von (E,Z)-7,9-Dodecandienyl-1-acetat nach Waschen und Verdampfen der organischen Lösungsmittel.

12. Verfahren nach Anspruch 11, **dadurch gekennzeichnet, dass** das Lösungsmittel S2 aus der Gruppe bestehend aus THF, MeTHF, Methylcyclohexan (MeCy), Diethylether, Methylterbutylether, 1,2-Dimethoxyethan und Mischungen davon ausgewählt ist.

13. Verfahren nach den Ansprüchen 11 und 12, **dadurch gekennzeichnet, dass** die Temperatur T3 zwischen 0 °C und 60 °C liegt.

14. Verfahren nach den Ansprüchen 11 bis 13, **dadurch gekennzeichnet, dass** das Katalysatorsystem Cat aus der Gruppe bestehend aus Eisentrihalogeniden, Eisentriacetat, Eisentriacetylacetonat, Eisennitrat (III), Eisenphosphat (III), gegebenenfalls in Gegenwart von Liganden ausgewählt aus Phosphor- oder Stickstoffliganden, ausgewählt wird.

15. Verfahren nach einem der Ansprüche 1 bis 14, **dadurch gekennzeichnet, dass** X' und X", die gleich oder verschieden sind, ein Halogen bezeichnen, das aus Chlor oder Brom ausgewählt ist, und dass das Acetylierungsmittel aus Acetoylhalogeniden, Essigsäureanhydrid und Alkylacetat ausgewählt ist.

## Claims

1. A compound of general formula 1 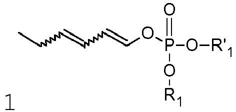 where R1 and R'1, identical or different, designate an alkyl or aryl group.

2. The compound according to claim 1 wherein the alkyl group is chosen from linear or branched C1-C6 alkyls and the aryl group is chosen from among phenyl, benzyl, mesityl or tolyl.

3. Use of a compound 1 according to one of claims 1 to 2, for the synthesis of a diene compound of the general formula: 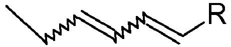 where R represents the radical of the compound onto which the conjugated diene unit is grafted.

4. Use of a compound 1 according to one of claims 1 to 2, for the synthesis of pheromones comprising at least one conjugated radical of the formula:
CH₃-CH₂-CH=CH=CH-

5. Use of a compound 1 according to one of claims 1 to 2 as an intermediate for the synthesis of (E,Z)-7,9-dodecadienyl-1-acetate.

6. A method for preparation of compound 1 comprising the following steps:
- provision of 2-hexenal in an appropriate solvent S1,
- addition of a weakly nucleophilic strong base at a temperature T1 comprised between -78°C and 25°C in order to form enolate,
- addition, at a temperature T2, identical to or different from T1, comprised between -78°C and 25°C, of a halophosphate of formula:
X-P(O)(OR1)(OR1')
where X is a halogen and R1 and R'1 designate identical or different groups chosen from among a linear or branched C1-C6 alkyl, an aryl such as phenyl, benzyl, mesityl or tolyl,
- recovery of compound 1 after washing and drying of the organic phase.

7. The method according to claim 6 **characterized in that** solvent S1 is chosen from the group consisting of tetrahydrofuran (THF), methyl tetrahydrofuran (MeTHF), tetramethylethylenediamine (TMEDA), tetrahydropyran, dimethoxyethane (DME), diethyl ether, methyl tert-butyl ether, highly polar solvents such as dimethylformamide (DMF), *N*-methylpyrrolidinone (NMP), N,N'-dimethyl propylene urea (DMPU), methylcyclohexane (MeCy), alkanes of fewer than 8 carbon atoms, aromatic solvents such as toluene and mixtures thereof.

8. The method according to one of claims 6 or 7, **characterized in that** temperatures T1 and T2, identical or different, are comprised between -40°C and 15°C.

9. The method according to one of claims 6 to 8, **characterized in that** temperatures T1 and T2, identical or different, are comprised between -20°C and 0°C.

10. The method according to claims 6 to 9, **characterized in that** the weakly nucleophilic strong base is chosen from the group consisting of sodium or potassium terbutanolate, sodium or potassium diisopropylamide, sodium or potassium hexamethyldisilyl azane, 1,5-diazabicyclo[4.3.0]non-5-ene (DBN), or 1,8-diazabicyclo[5.4.0]undec-7-ene (DBU).

11. A method for preparation of (E,Z)-7,9-dodecadienyl-1-acetate comprising the following steps:
- provision of product 1 in a solvent S2,
- addition of a catalytic system containing at least one iron atom at oxidation degree III,
- addition, at a temperature T3 comprised between -20° and 60°C, of the compound of general formula X'Mg-(CH₂)₆-OMgX" where X' and X", identical or different, designate a halogen,
- addition of an acetylation agent,
- recovery of (E,Z)-7,9-dodecadienyl-1-acetate after washing and evaporation of organic solvents.

12. The method according to claim 11, **characterized in that** solvent S2 is chosen from among the group consisting of THF, MeTHF, methylcyclohexane (MeCy), diethyl ether, methyl tert-butyl ether, 1,2-dimethoxyethane and mixtures thereof.

13. The method according to claims 11 and 12, **characterized in that** temperature T3 is comprised between 0°C and 60°C.

14. The method according to claims 11 to 13, **characterized in that** the catalytic system Cat is chosen from the group consisting of iron trihalides, iron triacetate, iron tris-acetylacetonate, iron (III) nitrate, iron (III) phosphate optionally in the presence of ligands chosen from phosphorus or nitrogen ligands.

15. The method according to one of claims 1 to 14, **characterized in that** X' and X", identical or different, designate a halogen chosen from among chlorine or bromine, and the acetylation agent is chosen from among the acetoyl halides, acetic anhydride and alkyl acetate.
